(19) Europäisches Patentamt
European Patent Office
Office européen des brevets

(11) Publication number: **0 357 332 A2**

# (12) EUROPEAN PATENT APPLICATION

(21) Application number: **89308551.4**

(22) Date of filing: **23.08.89**

(51) Int. Cl.[5]: **A 61 K 37/64**
A 61 K 37/02, A 61 K 31/00,
C 07 K 5/02

(30) Priority: **24.08.88 US 235841 28.03.89 US 328644**

(43) Date of publication of application:
**07.03.90 Bulletin 90/10**

(84) Designated Contracting States:
**AT BE CH DE ES FR GB IT LI LU NL SE**

(71) Applicant: **MERCK & CO. INC.**
**126, East Lincoln Avenue P.O. Box 2000**
**Rahway New Jersey 07065-0900 (US)**

(72) Inventor: **Sigal, Irving S.**
**Deceased**
**Deceased (US)**

**Huff, Joel R.**
**738 Bergey Mill Road**
**Lederach, PA 19450 (US)**

**Darke, Paul L.**
**434 West Walnut Street**
**North Wales, PA 19454 (US)**

(74) Representative: **Hesketh, Alan, Dr. et al**
**European Patent Department Merck & Co., Inc. Terlings Park Eastwick Road**
**Harlow Essex, CM20 2QR (GB)**

Claim for the following Contracting State: ES

## (54) Renin inhibitors useful for the treatment of aids by inhibition of HIV protease.

(57) Certain renin inhibitors are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 357 332 A2

## Description

# RENIN INHIBITORS USEFUL FOR THE TREATMENT OF AIDS BY INHIBITION OF HIV PROTEASE

The present invention is concerned with compounds which inhibit the protease encoded by human immunodeficiency virus (HIV) or pharmaceutically acceptable salts thereof and are of value in the prevention of infection by HIV, the treatment of infection by HIV, and the treatment of the resulting acquired immune deficiency syndrome (AIDS).

It also relates to pharmaceutical compositions containing the compounds and to a method of use of the present compounds and other agents for the treatment of AIDS.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Interruption of this processing appears to prevent the production of normally infectious virus. For example, Crawford, S. et al., J. Virol.,53, 899, 1985, demonstrated that genetic deletion mutations of the protease in murine leukemia virus which prevent processing of precursor structural proteins results in non-infectious viral particles. Unprocessed structural proteins also have been observed in clones of non-infectious HIV strains isolated from human patients. These results suggest that inhibition of the HIV protease represents a viable method for the treatment of AIDS or the prevention of infection by HIV.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)]. Applicants demonstrate that the compounds of this invention are inhibitors of HIV protease.

Compounds of this invention are disclosed broadly as renin inhibitors in U.S. Patent 4,661,473; Evans, B.E. et al., J. Org. Chem., 50, 4615, (1985) and Evans, B.E. et al., "A Stereocontrolled Synthesis of Hydroxyethylene Dipeptide Isosteres," Proc. Am. Pept. Syrup., 9, 743-6(198 ). Applicants demonstrate that renin inhibitors are inhibitors of HIV protease. The compounds of the present invention are useful in the prevention of infection by HIV, the treatment of infection by HIV, and the treatment of AIDS, ARC and diseases of similar etiology.

## BRIEF DESCRIPTION OF THE INVENTION

Compounds of formula I, as herein defined, are disclosed. These compounds are useful in the inhibition of HIV protease, the prevention of infection by HIV, the treatment of infection by HIV, and the treatment of AIDS, either as compounds, pharmaceutically acceptable salts, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

## ABBREVIATIONS

| Designation | Amino Acid/Residue |
|---|---|
| Ala | D- or L-alanine |
| Arg | D- or L-arginine |
| Cal (Cha) | β-cyclohexylalanine |
| Cys | D- or L-cysteine |
| Gly | glycine |
| Glu | D- or L-glutamic acid |
| His | D- or L-histidine |
| Ile | L-isoleucine |
| Leu | D- or L-leucine |
| Lys | D- or L-lysine |
| Met | D- or L-methionine |
| Nle | L-norleucine |
| Nva | L-norvaline |
| Orn | D- or L-ornithine |
| Phe | D- or L-phenylalanine |
| Pro | D- or L-proline |
| Sar | sarcosine (N-methylglycine) |
| Ser | D- or L-serine |
| Sta | statine, (3S,4S)-4-amino-3-hydroxy-6-methylheptanoic acid |
| Thr | D- or L-threonine |
| Trp | D- or L-tryptophan |
| Tyr | D- or L-tyrosine |
| Val | L-valine |

| | Protecting Group |
|---|---|
| Ac | Acetyl |
| BOC (Boc) | t-butyloxycarbonyl |
| BOM | benzyloxymethyl |
| CBZ (Cbz) | benzyloxycarbonyl(carbobenzoxy) |
| DNP | 2,4-dinitrophenyl |
| IPOC | isopropoxycarbonyl |
| OMe | methyl ether (methoxy), except when it immediately follows an amino acid residue abbreviation and it represents methyl ester |
| OEt | ethoxy, except when it immediately follows an amino acid residue abbreviation it and represents ethyl ester |

| Designation | Activating Group |
|---|---|
| HBT(HOBT) | 1-hydroxybenzotriazole |
| OMs | hydrate methane sulfonyloxy |

| | Condensing Agent |
|---|---|
| DCCI (DCC) | dicyclohexylcarbodiimide |
| DPPA | diphenylphosphorylazide |

| | Reagent |
|---|---|
| AMP | 4-aminomethylpyridine |
| $(BOC)_2O$ | di-t-butyl dicarbonate |
| DEAD | diethyl azodicarboxylate |
| TEA | triethylamine |
| TFA | trifluoroacetic acid |

| | Coupling Reagents |
|---|---|
| BOP reagent | benzotriazol-1-yloxytris-(dimethyl-amino)phosphonium hexafluorophosphate |
| BOP-Cl | bis(2-oxo-3-oxazolidinyl) phosphinic chloride |
| DSO | N,N'-disuccinimidyl oxalate |
| | **Solvent** |
| AcOH (HOAc) | acetic acid |
| DMF | dimethylformamide |
| EtOAc (E) | ethyl acetate |
| $Et_2O$ | ether |

## DETAILED DESCRIPTION OF THE INVENTION AND PREFERRED EMBODIMENTS

This invention is concerned with the use of compounds of Formula I, combinations thereof, or pharmaceutically acceptable salts thereof, in the inhibition of HIV protease, prevention or treatment of infection by HIV and the treatment of the resulting acquired immune deficiency syndrome (AIDS). Compounds of formula I are defined as follows:

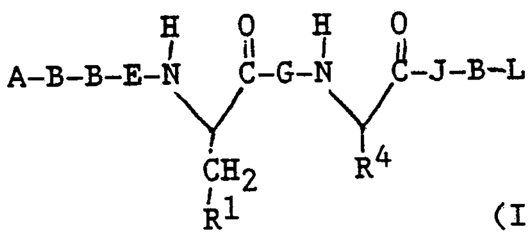

(I.)

wherein:
A is hydrogen; or

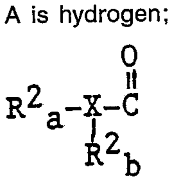

where
X is -O-; S; -CH-; -NH-CH-; and
$R^2_a$ and $R^2_b$ may be the same or different and are hydrogen; $C_{1-4}$-alkyl; aryl; $C_{3-7}$-cycloalkyl; or $C_{3-7}$-cycloalkyl or aryl substituted with up to five members independently selected from the group consisting of $C_{1-8}$-alkyl, trifluoromethyl, hydroxy, $C_{1-4}$-alkoxy, and halo; except that where X is -O-, only one of $R^2_a$ or $R^2_b$ is present;
B is absent; glycyl; prolyl; sarcosyl; or

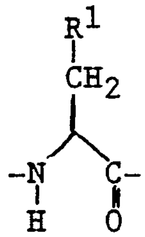
,

where $R^1$ is hydrogen; $-CONH_2$; -COOH; $C_{1-4}$-alkyl; hydroxy $C_{1-4}$-alkyl; $C_{1-4}$-alkyl substituted with carboxy; aryl; aryl substituted with up to three members selected from the group consisting of $C_{1-4}$-alkyl, hydroxy, $C_{1-4}$-alkoxy, and halo; indolyl; imidazolyl; amino $C_{2-4}$-alkyl;
E is absent; or

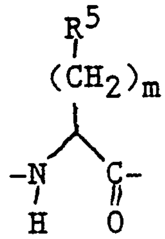
,

where m is 1 to 4; and
$R^5$ is (1) hydrogen;
(2) $C_{1-4}$-alkyl;
(3) $C_{1-4}$-alkyl substituted with carboxy;
(4) aryl;
(5) aryl substituted with up to three members selected from the group consisting of $C_{1-4}$-alkyl, hydroxy, $C_{1-4}$-alkoxy, and halo;
(6) $R^5$ is indolyl; or
(7) imidazolyl;
G is

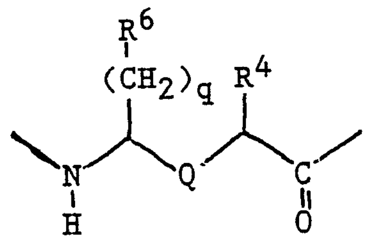

where q is 1 to 4;
$R^4$ is hydrogen; or

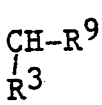
,

where
$R^3$ is hydrogen; $C_{1-4}$-alkyl; aryl; aryl $C_{1-4}$-alkyl; $C_{1-4}$-alkyl or aryl or aryl $C_{1-4}$-alkyl substituted with up to three members selected from the group consisting of $C_{1-4}$-alkyl, carboxy, hydroxy, $C_{1-4}$-alkoxy, and halo; or indolyl;
$R^9$ is hydrogen; $C_{1-4}$-alkyl; hydroxy; carboxy; or $C_{3-7}$-cycloalkyl;
$R^6$ is $C_{3-6}$-alkyl; $C_{3-7}$-cycloalkyl; aryl; or $C_{3-7}$-cycloalkyl or aryl substituted with up to three members selected from the group consisting of $C_{1-4}$-alkyl, hydroxy, $C_{1-4}$-alkoxy, and halo; and

Q is 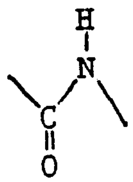 ; 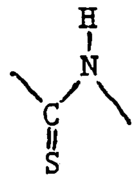 ; 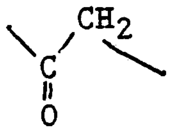 ;

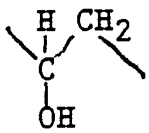 ; 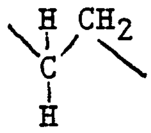 ; 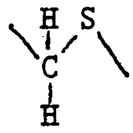 ;

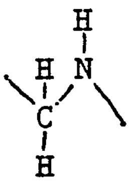 ; 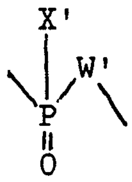 ; 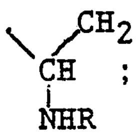 ; 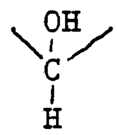 ;

wherein X′ is hydroxy, amino, or mono-or di-$C_{1-4}$-alkyl amino; and W′ is absent, or $-CH_2-$; or

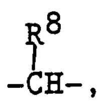,

where $R^8$ is hydrogen or $C_{1-3}$-alkyl;

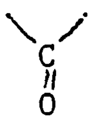 ; 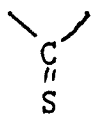 ; or 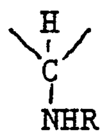 ;

where R is hydrogen; $C_{1-4}$-alkyl; or formyl;
J is absent; or
(1) $-Y-(CH_2)_n-R^7$
where
Y is -NH-;
n is 0 to 5; and
$R^7$ is hydrogen; hydroxy; $C_{1-4}$-alkyl; $C_{3-7}$-cycloalkyl; carbocyclic; aryl; carbocyclic or aryl substituted with up to five members independently selected from the group consisting of $C_{1-6}$-alkyl, $C_{1-6}$-alkyl, hydroxy, $C_{1-4}$-alkoxy, amino, mono- or di- $C_{1-4}$-alkylamino, and halo; amino; mono-, di-, or tri-$C_{1-4}$-alkylamino; amino substituted with heterocycle; guanidyl; heterocycle; heterocycle mono-$C_{1-4}$-alkylamino or heterocycle substituted with up to five members independently selected from the group consisting of $C_{1-6}$-alkyl, hydroxy, $C_{1-4}$-alkoxy, halo, amino, and mono- or di-$C_{1-4}$-alkylamino;

(2) 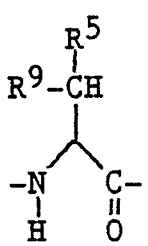 , where $R^5$ and $R^9$ are defined above; or

(3) (a) 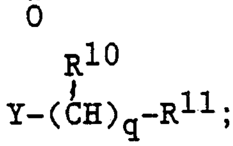; (b) 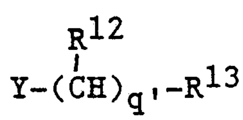; or

(c) 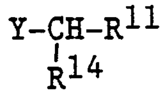

where
Y is -NH-;
q is 1-5;
q′ is 0-5;
$R^{10}$ is hydrogen; hydroxy; $N(R')_2$, where R′ may be the same or different and is hydrogen or $C_{1-4}$-alkyl; guanidyl; or $N(R')_3^{\oplus}A^{\ominus}$, where R′ is as defined above, and $A^{\ominus}$ is a counterion; provided that at least one $R^{10}$ is not hydrogen;
$R^{11}$ is $C_{1-4}$-alkyl; $C_{3-7}$-cycloalkyl; aryl; aryl substituted with up to three members independently selected from the group consisting of $C_{1-6}$-alkyl, hydroxy, $C_{1-4}$-alkoxy, amino, mono- or di-$C_{1-4}$-alkylamino, amino $C_{1-4}$-alkyl, mono-, di-, or tri-$C_{1-4}$-alkylamino-$C_{1-4}$-alkyl, carboxy, carboxy ester or amide, carboxy-$C_{1-4}$-alkyl, carboxy-$C_{1-4}$-alkyl ester or amide; carboxy, ester or amide; sulfo; heterocycle; or heterocycle substituted with up to five members independently selected from the group consisting of hydroxy, $C_{1-4}$-alkoxy, halo, amino, and mono- or di-$C_{1-4}$-alkylamino;
$R^{12}$ is hydrogen; or carboxy, ester or amide;
$R^{13}$ is carboxy, ester or amide; sulfo; or aryl substituted with up to three members selected from the group consisting of amino-$C_{1-4}$-alkyl, mono-, di-, or tri-$C_{1-4}$-alkylamino-$C_{1-4}$-alkyl, carboxy, carboxy ester or amide,
$R^{14}$ is carboxy, ester or amide;
L is absent; OR″; NHR″; or $N(R'')_2$, where R″ may be the same or different and is hydrogen or $C_{1-4}$-alkyl; or pharmaceutically acceptable salts thereof.

In the peptides of the present invention, amino acids adjoining G, as well as the B, E, G and J components may have asymmetric centers and occur as racemates, racemic mixtures and as individual diastereomers, with all isomeric forms generally being included in the present invention. In particular, asymmetric carbon atoms in the G substituent has either an S or an R configuration.

In one embodiment of this invention, A of Formula I is t-butyloxycarbonyl, ethoxycarbonyl, or t-butylacetyl. B is absent. $R^5$ is $C_{1-4}$-alkyl or aryl. $R^1$ is $C_{1-4}$-alkyl, hydroxy, $C_{1-4}$-alkyl, or aryl. $R^6$ is $C_{3-7}$-cycloalkyl, aryl, or hydroxyaryl. Q is

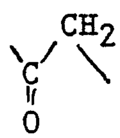 ; 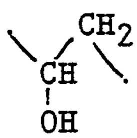 ; 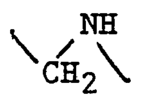 ; 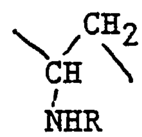 ; or

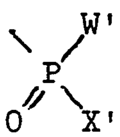 . $R^9$ is H. $R^3$ is $C_{1-4}$-alkyl, aryl, or substituted aryl. J is defined as in (1) or (2). $R^7$ is $C_{1-4}$-alkyl, aryl, pyridyl or pyridylamino. L is NHR″.

In another embodiment of this invention, A of Formula I is t-butyloxycarbonyl. $R^5$ is aryl. $R^1$ is aryl. $R^6$ is $C_{3-7}$-cycloalkyl, aryl or hydroxyaryl. Q is

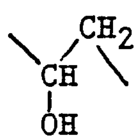 or 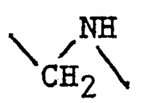 or 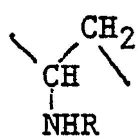 ;

$R^3$ is aryl.

The preferred embodiment encompasses Compounds A, B, and C.

A:

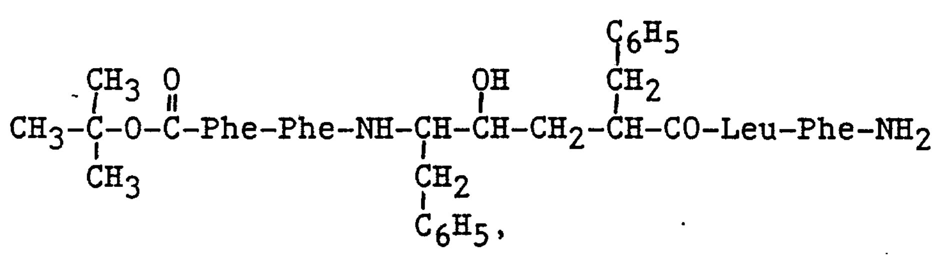

N′-(1,1-Dimethylethoxycarbonyl)-Phe-Phe-5(S)-Amino-4(S)-Hydroxy-6-Phenyl-2(R or S)-(Phenylmethyl)Hexanoyl-Leu-Phe-Amide;

B:

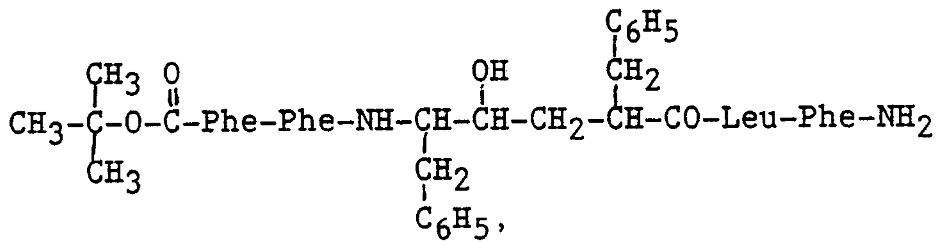

N′-(1,1-Dimethylethoxycarbonyl)-Phe-Phe-5(S)-Amino-4(S)-Hydroxy-6-Phenyl-2(S or R)-(Phenylmethyl)Hexanoyl-Leu-Phe-Amide;

C:

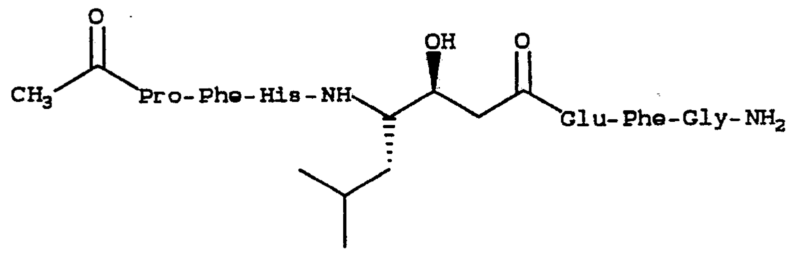

N′-Acetyl-Pro-Phe-His-4(S)-Amino-3(S)-Hydroxy-6-methylheptanoyl-Glu-Phe-Gly-Amide.

Other preferred compounds include the following:

N′-(1,1-Dimethylethoxycarbonyl)-Phe-Phe-5(S)-amino-4(S)-hydroxy-6-phenyl-2(R)-(phenylmethyl)hexanoyl-N-(phenylmethyl)-Leu -amide,

N′-(1,1-Dimethylethoxycarbonyl)-Phe-Phe-5(S)-amino-4(S)-hydroxy-6-phenyl-2(R)-(phenylmethyl)hexanoyl-Leu-amide,

N′-(1,1-Dimethylethoxycarbonyl)-Asn-5(S)-amino-4(S)-hydroxy-6-phenyl-2(R)-(phenylmethyl)hexanoyl-Leu-Phe-amide,

Asn-5(S)-amino-4(S)-hydroxy-6-phenyl-2(R)-(phenylmethyl)hexanoyl-Leu-Phe-amide,

N′-(1,1-dimethylethoxycarbonyl)-Asp-5(S)-amino-4(S)-hydroxy-6-phenyl-2(R)-(phenylmethyl)hexanoyl-N-(phenylmethyl)-Ile-amide,

5(S)-[(1,1-dimethylethoxycarbonyl)-Asp]amine-4(S)-hydroxy-6-phenyl-2(R)-(phenylmethyl)-N-(Cis-2(R)-hydroxy-1(S)-indanyl)hexanamide,

5(S)-Asp-amino-4(S)-hydroxy-6-phenyl-2(R)-(phenylmethyl)-N-(cis-2(R)-hydroxy-1(S)-indanyl)hexanamide,

or pharmaceutically acceptable salt thereof.

When any variable (e.g., aryl, heterocycle, R, R′, $R^1$, $R^2$, $R^3$, $R^4$,$R^5$, $R^6$, $R^7$, $R^{10}$, $R^{11}$, $R^{12}$, $R^{14}$, $A^\ominus$, m, q, etc.) occurs more than one time in any constituent or in formula I, its definition on each occurrence is independent of its definition at every other occurrence. Such independent definitions are in some instances emphasized, e.g. $R^2{}_a$ and $R^2{}_b$, each being $R^2$. Combinations of substituents and/or variables are permissible only if such combinations result in stable compounds.

As used herein, except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; "carboxy" is -COOH; and "cycloalkyl" is intended to include saturated ring groups, such as cyclopropyl, cyclobutyl, cyclopentyl, cyclohexyl (Cyh) and cycloheptyl. "Arylalkyl" represents aryl groups as herein defined which are attached through a straight- or branched- chain alkyl group of specified number of carbon atoms, such as, for example, benzyl, phenethyl, 3,3-diphenylpropyl, 3-indolymethyl, and the like. "Halo", as used herein, means fluoro, chloro, bromo and iodo; and "counterion" is used to represent a small, single negatively-charged species, such as chloride, bromide, hydroxide, acetate, perchlorate, nitrate, benzoate, maleate, tartrate, hemitartrate, benzene sulfonate, and the like.

As used herein, with exceptions as noted, "aryl" is intended to mean phenyl (Ph) or naphthyl, which is optionally substituted by from one- to three- members independently selected from the group consisting of $C_{1-8}$-alkyl, amino, mono- or di-$C_{1-4}$-alkylamino, amino-$C_{1-4}$-alkyl, phenyl-$C_{1-4}$-alkyl, hydroxy-$C_{1-4}$-alkyl, di-$C_{1-4}$-alkylamino-$C_{1-4}$-alkyl, guanidyl, guanidyl-$C_{1-4}$-alkyl, hydroxyl, $C_{1-4}$-alkoxy; $CF_3$, halo, CHO, $CO_2H$, $CO_2$-$C_{1-3}$-alkyl, $CO_2$-$C_{1-4}$-alkoxy, $CO_2R^7$, NR′R′, wherein $R^7$ and R′ are defined in Formula I, or $N(R')_3{}^\oplus A^\ominus$, wherein $A^\ominus$ is a counterion, as defined herein. "Carbocyclic" is intended to mean any stable 5- to 7-membered carbon ring or 7- to 10-membered bicyclic carbon ring, any of which may be saturated or partially unsaturated.

The term heterocycle, as used herein except where noted, represents a stable 5- to 7-membered mono- or bicyclic or stable 7- to 10-membered bicyclic heterocyclic ring which is either saturated or unsaturated, and

which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. Fully-saturated heterocyclic substituents are preferred and those in which nitrogen is the heteroatom are also preferred, with those containing a single nitrogen atom being particularly preferred. The heterocyclic ring may be attached at any heteroatom or carbon atom which results in the creation of a stable structure. Examples of such heterocyclic elements include piperidyl, piperidinyl, piperazinyl, 2-oxopiperazinyl, 2-oxopiperidinyl, 2-oxopyrrolodinyl, 2-oxoazepinyl, azepinyl, pyrryl, pyrrolinyl, 4-piperidonyl, pyrrolidinyl, pyrazolyl, pyrazolinyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, benzothiazolyl, benzoxazolyl, furyl, thienyl, benzothienyl, thiamorpholinyl, thiamorpholinyl sulfoxide, and thiamorpholinyl sulfone. The heterocyclic moiety is further optionally-substituted, in a manner such that carbon atoms attached to a heteroatom are not directly substituted by a heteroatom, by from one- to four- members independently selected from the group consisting of hydroxyl, thiol, $C_{1-6}$-alkyl, $CF_3$, $C_{1-4}$-alkoxy, halo, aryl, aryl-$C_{1-4}$-alkyl, amino, mono- or di-$C_{1-4}$-alkylamino, amino-$C_{1-4}$-alkylamino, amino-$C_{1-4}$-alkyl, hydroxy-$C_{1-4}$-alkyl, di-$C_{1-4}$-alkylamino-$C_{1-4}$-alkyl, guanidyl, guanidyl-$C_{1-4}$-alkyl, CHO, $CO_2H$, $CO_2R^7$, NR′R′,
wherein aryl and R′ are as defined in Formula I, or $N(R')_3^{\oplus}A^{\ominus}$,
wherein R′ is as defined in Formula I and $A^{\ominus}$ is a counterion, as defined herein, with aryl-$C_{1-4}$-alkyl being preferred, or a spiro quaternary N bicyclic may be formed.

The pharmaceutically-acceptable salts of the peptides of Formula I (in the form of water- or oil-soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium slats, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

HIV protease inhibitors of Formula I may be prepared in accordance with well-known procedures for preparing peptide analogs from their constituent amino acids or analogs thereof.

In general, once the G substituent is made, the rest of the synthesis follows the principle and practice of amide bond formation by the coupling methods of either solution-phase or solid-phase peptide synthesis. The addition and removal of one or more protecting groups is also typical practice.

Other embodiments of the present invention include the following compounds:

1. 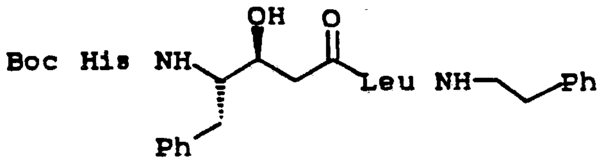

2. 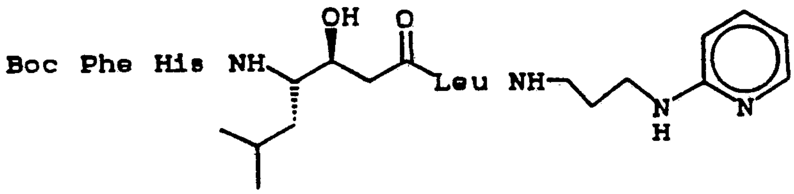

3. 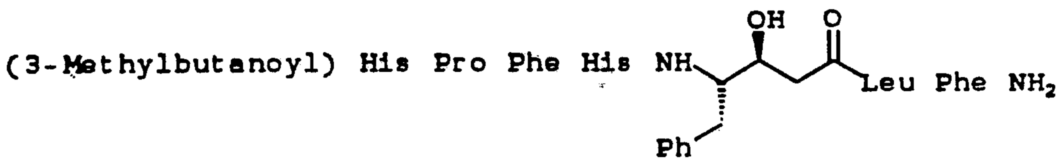

4. 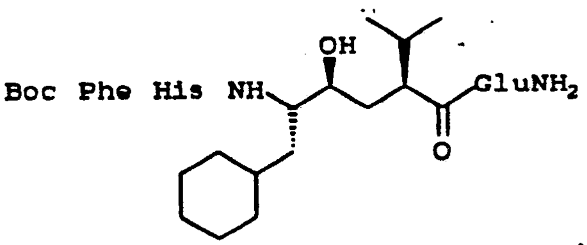

5. 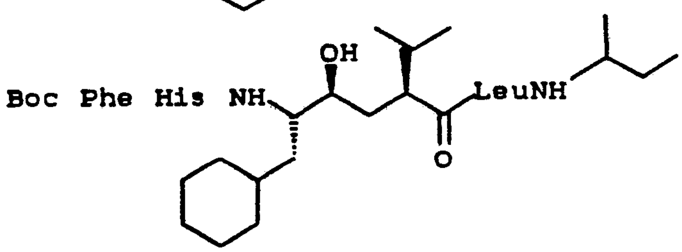

6. 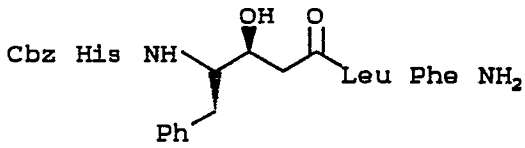

7. 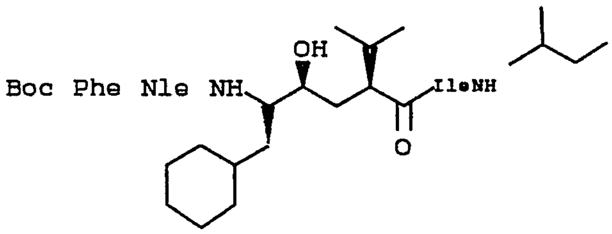

8. Ac Phe His Sta Glu Phe $NH_2$

9. 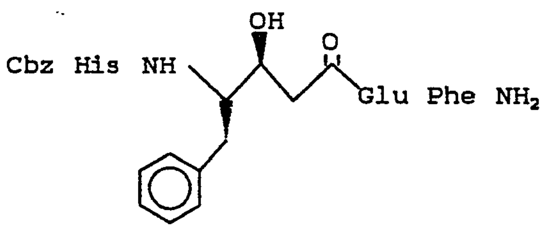

10. 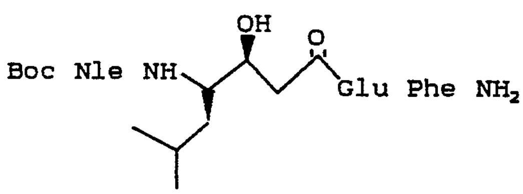

11. 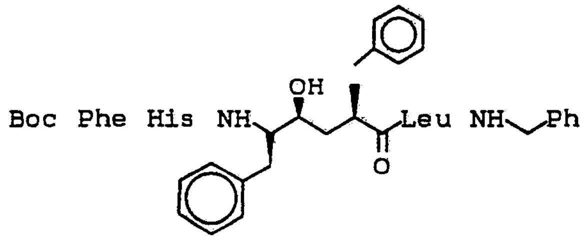

12. 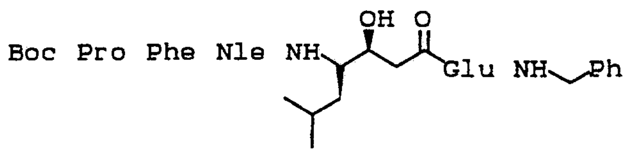

13. 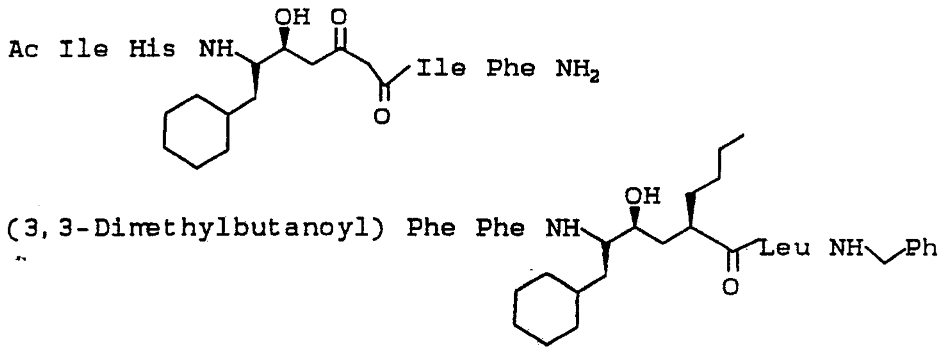

14.

15. 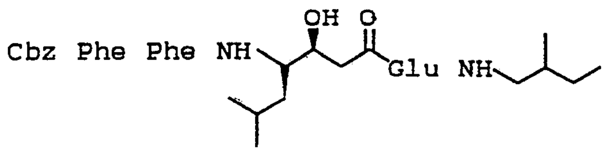

16. 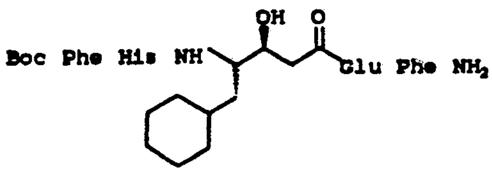

17. 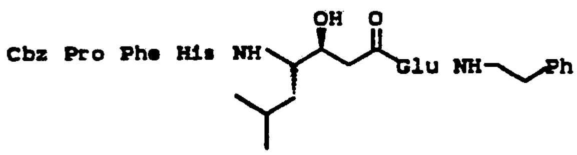

18. 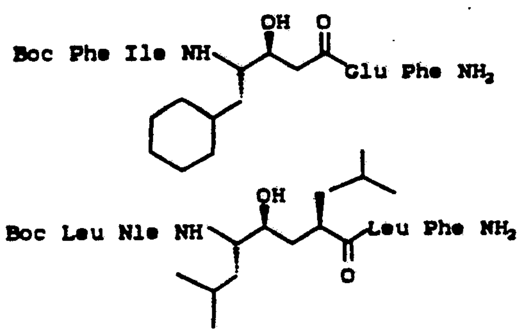

19.

20. 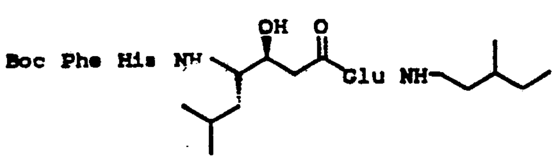

Structures and abbreviations for the G components of the inhibitors of the present invention include:

1. 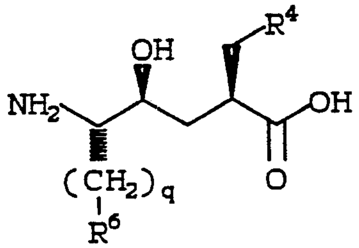 ,

a hydroethylene dipeptide isostere prepared, for example, by an intermediate lactone according to Evans, B.E. et al J. Org. Chem. 50, 4615(1985) or Kempf, D.J. J. Org. Chem. 51, 3921(1986). Synthetic routes to similar peptide bond isosteres, such as

2. 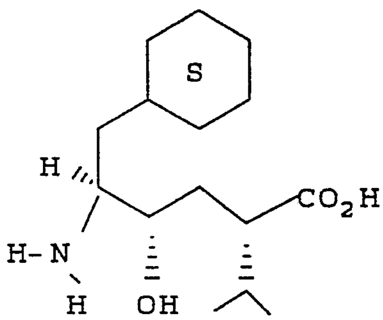

$Cal[CH(OH)CH_2]Val$,

are readily available. The intermediate BOC-Cal[CH(OH)CH2]Val-OH lactone is obtained from intermediates prepared using methods described by P. Buhlmayer et al, in Published European Patent Application 184,550-A2. Other synthetic routes to peptide bond isosteres of like character are described in the following:

a. Szelke et al, in Peptides, Structure and Function. Proceedings of the Eighth American Peptide Symp(ed. V.J. Hruby and D.H. Rich) pp. 579-82, Pierce Chemical Co., Rockford, IL;

b. D.T. Pals et al in European Patent Appln. 173,481-A2;

c. A.H. Fray et al, J. Org. Chem., 51, 4828-4833 (1986); and

d. M. Szelke et al, PCT Int. Appl. WO 84 03,044.

Structures and abbreviations for other G components of the inhibitors of the present invention include:

3. 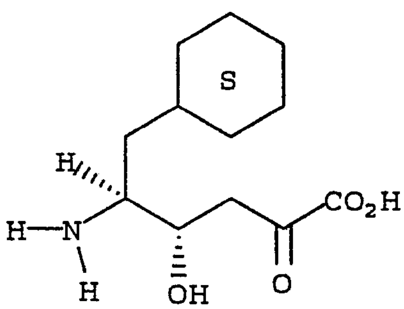

ACHPA

with BOC-ACHPA-OEt being prepared by the method described by Boger et al., J. Med. Chem., 28, 1779-1790 (1985);

4.

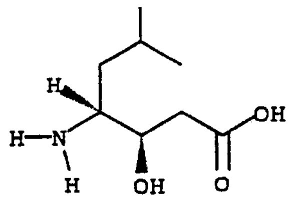

Statine (Sta)

with BOC-Sta-OEt being prepared in accordance with the procedure described by Rich et al., J. Org. Chem., 43, 3624 (1978);

5.

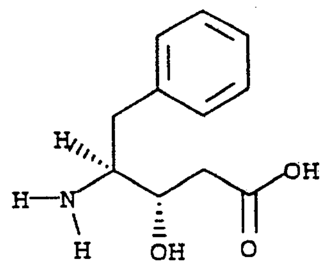

AHPPA

with BOC-AHPPA-OEt being prepared as described by Rich et al., J. Org. Chem., 23, 27-33 (1980);

6.

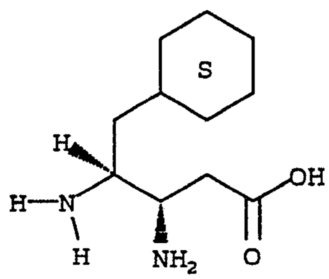

AmACHPA

7.

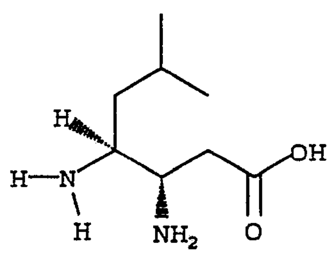

AmSta

with BOC-AmSta(CBZ)-OH being prepared as shown in the following Scheme, and BOC-AmACHPA(CBZ)-OH being prepared as illustrated in the Scheme by substituting BOC-ACHPA-OEt for BOC-Sta-OEt:

Synthesis of protected 3-amino-3-deoxy-(3S, 4S)-statine:

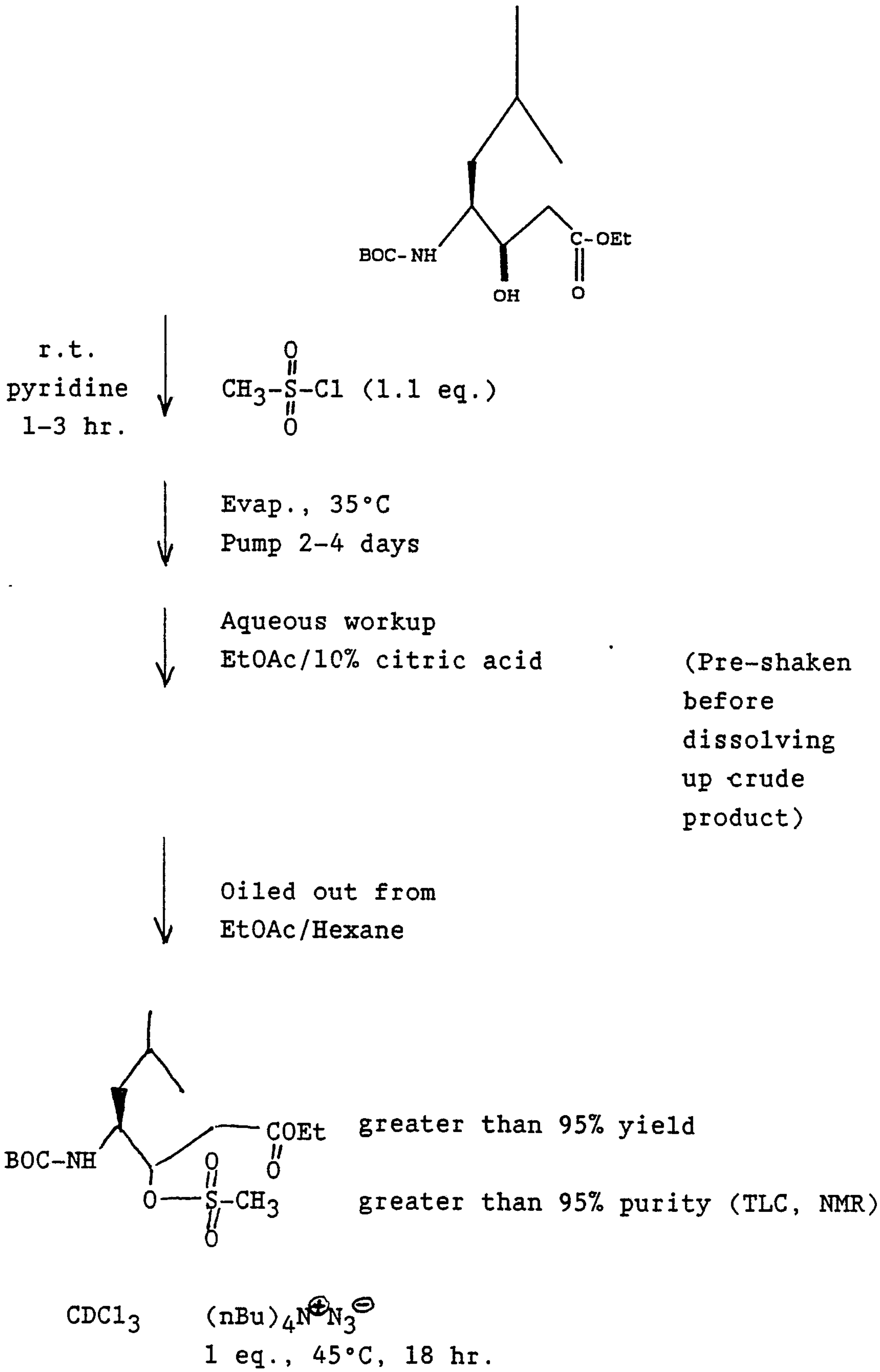
r.t.
pyridine
1-3 hr.
(1.1 eq.)
Evap., 35°C
Pump 2-4 days
Aqueous workup
EtOAc/10% citric acid
(Pre-shaken before dissolving up crude product)
Oiled out from
EtOAc/Hexane
greater than 95% yield
greater than 95% purity (TLC, NMR)
CDCl3
1 eq., 45°C, 18 hr.

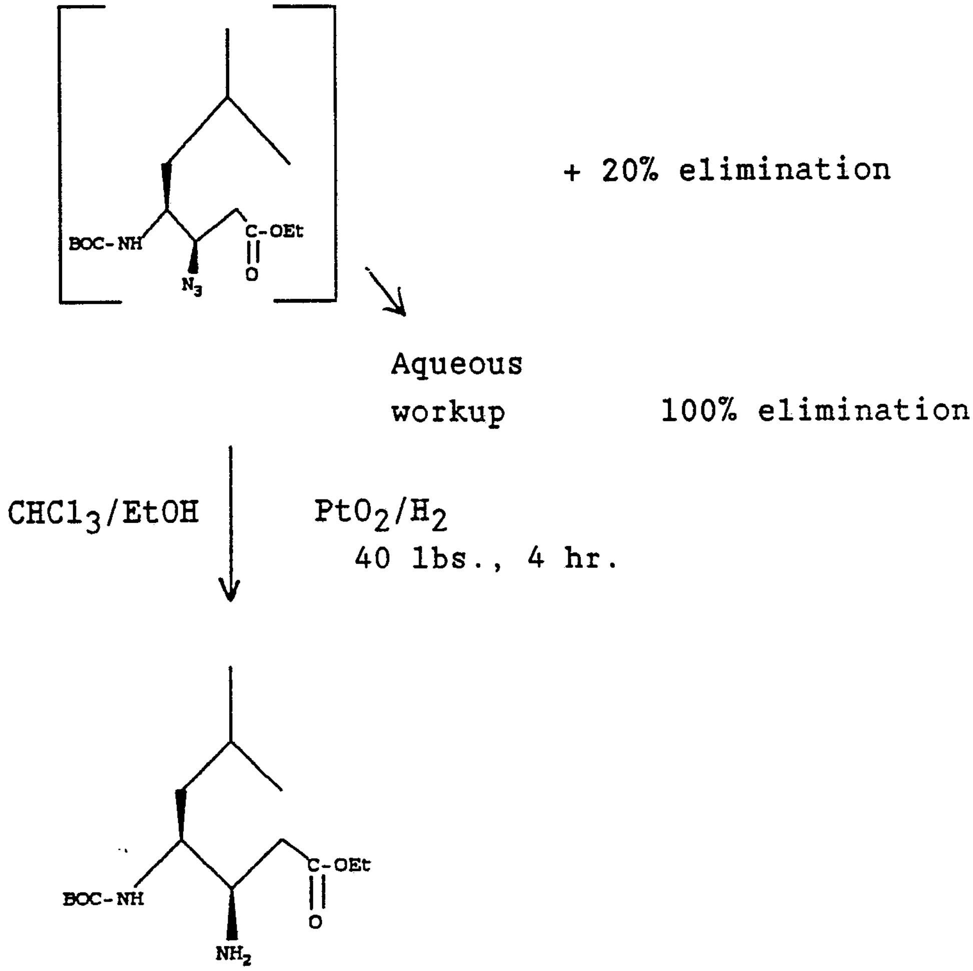


the amine is isolated by extraction into weak acid. Protection of the amine function is as follows:

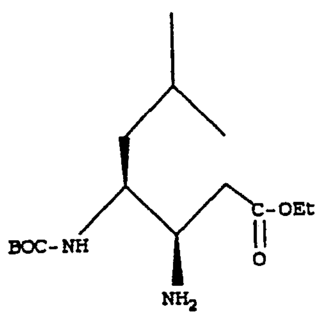

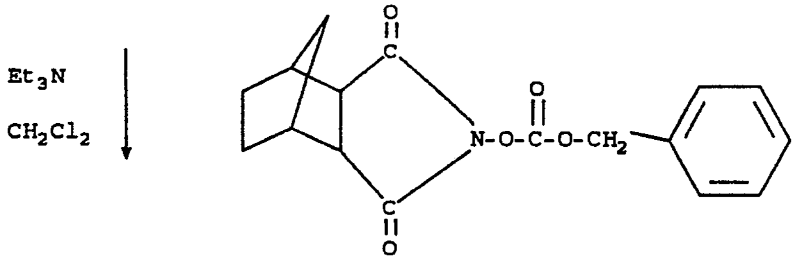


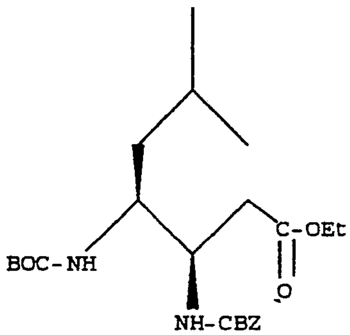

+ excess CBZ reagent

(predominantly R at position 3 as shown).

Base hydrolysis gives the free acid for incorporation into the synthesis of the peptide of Formula I; or alternatively, this material may be prepared as described by Jones et al, in Peptides, Structure and Function. Proceedings of the Ninth American Peptide Symposium (eds. C. M. Deber, V.J. Hruby and K. D. Kopple) pp. 759-62, 1985, Pierce Chemical Co., Rockford, IL.; Arrowsmith et al, J. Chem. Soc. Chem. Commun. 755-7 (1986); and Raddatz et al, Published Eur. Pat. Appl. 161,588;
with efficient methods for preparing the 2-substituted statine component G (such as

8. 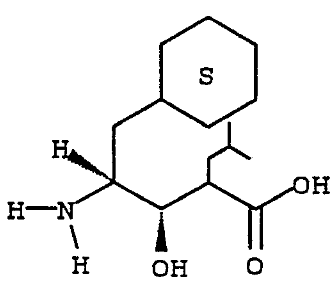

(2-isobutyl)ACHPA

in a suitably protected form being described in Pub. Eur. Pat Appln. 157,409 (with other pertinent references including D. Veber et al, Biochem. Soc. Trans., 12, 956-959 (1984) and H. Stein et al, Fed. Proc. 45, 869, Abstract No 4151 (1986).

Amide couplings used to form the compounds of this invention are typically performed by the carbodiimide method with reagents such as dicyclohexylcarbodiimide, or N-ethyl-N′-(3-dimethylaminopropyl) carbodiimide. Other methods of forming the amide or peptide bond include, but are not limited to synthetic routes via an acid chloride, azide, mixed anhydride or activated ester. Typically, solution phase amide couplings with or without peptide fragments are performed, but solid-phase synthesis by classical Merrifield techniques may be employed instead.

The selection of protecting groups is, in part, dictated by particular coupling conditions, in part by the amino acid and peptide components involved in the reaction. Such amino-protecting groups ordinarily employed include those which are well known in the art, for example, urethane protecting substituents such as

benzyloxycarbonyl (carbobenzoxy), p-methoxycarbobenzoxy, p-nitrocarbobenzoxy, t-butyloxycarbonyl, and the like. It is preferred to utilize t-butyloxycarbonyl (BOC) for protecting the α-amino group, in part because the BOC protecting group is readily removed by relatively mild acids such as trifluoroacteic acid (TFA), or hydrogen chloride in ethyl acetate.

The OH group of Thr and Ser may be protected by the Bzl (benzyl) group and the ε-amino group of Lys may be protected by the IPOC group or the 2-chlorobenzyloxycarbonyl (2-Cl-CBZ) group. Treatment with HF or catalytic hydrogenation are typically employed for removal of IPOC or 2-Cl-CBZ.

The compounds of the present invention are useful in the inhibition of HIV protease and the prevention or treatment of infection by the human immunodeficiency virus (HIV) and the treatment of consequent pathological conditions such as AIDS. Treating AIDS, preventing infection by HIV or treating infection by HIV, is defined as including, but not limited to, treating a wide range of states of HIV infection: AIDS, ARC (AIDS related complex), both symptomatic and asymtomatic, and actual or potential exposure to HIV. For example, the compounds of this invention are useful in preventing infection by HIV after suspected past exposure to HIV by, e.g., blood transfusion, accidental needle stick, or exposure to patient blood during surgery.

In the present invention, compounds with asymmetric centers may occur as racemates, racemic mixtures and as individual diastereomers, with all isomeric forms of the compounds being included in the present invention.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination the severity of the particular condition, and the host undergoing therapy.

The present invention is also directed to combinations of the HIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the Table I [source: Marketletter, Nov. 30. 1987, pp. 26-27; Genetic Engineering News, Jan. 1988, Vol. 8, 23]:

TABLE I[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho- protein) | Advanced Viral Research | AIDS, ARC |
| RETROVIR (zidovudine; AZT) | Burroughs Wellcome | AIDS, advanced ARC |
| | | pediatric AIDS, KS, asympt HIV, less severe HIV, neurological in- volvement. |

| Drug Name | Manufacturer | Indication |
| --- | --- | --- |
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| | | |
| --- | --- | --- |
| ABPP (bropirimine) | Upjohn | Advanced AIDS, KS |
| AMPLIGEN (mismatched RNA) | DuPont HEM Research | ARC, PGL |
| (Anti-human alpha interferon antibody) | Advanced Biotherapy Concepts | AIDS, ARC, KS |

| Drug Name | Manufacturer | Indication |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| Drug Name | Manufacturer | Indication |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

C. Antibiotics

| Drug Name | Manufacturer | Indication |
|---|---|---|
| PENTAM 300 (pentamidine isethionate) | LyphoMed | PCP |

D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, immunomodulators, antibiotics or vaccines is not limited to the list in the above Table, but includes in principle any combination with any pharmaceutical composition useful for the treatment of AIDS.

## SYNTHESIS

The preparation and synthesis follows, in general, U.S. Patent 4,661,473; Evans, B.E. et al, J. Org. Chem., 50, 4615-4625, (1985) and Evans, B.E. et al., "A Stereocontrolled Synthesis of Hydroxyethylene Dipeptide Isosteres," Proc. Am. Pept. Symp., 9, 743-6(198), all herein incorporated by reference.

## EXAMPLE 1

Step A. 1-[1-(Boc-amino)-2-phenylethyl]oxirane as product.

Step B. 5-[1-(t-Boc-amino)-2-phenylethyl]-3-carbethoxydihydrofuran-2(3H)-one as product.

Step C. 5-[1-(t-Boc-amino)-2-phenylethyl]-3-carbetheoxy--(phenylmethyl)dihydrofuran-2(3H)-one as product.

Step D. 5-[1-(t-Boc-amino)-2-phenylethyl]-3-(phenylmethyl)dihydrofuran-2-(3H)-one as product.

Step E. 5-(t-Boc-amino)-4-[(tert-butyldimethylsilyl)oxy]-6-phenyl-2-(phenylmethyl)hexanoic Acid as product.

Step F. 5-(1-Amino-2-phenylethyl)-3-(phenylmethyl)dihydrofuran-2(3H)-one Hydrochloride as product.

Step G. N$^{\alpha}$-[5-[[N$^{\alpha}$-(t-Boc-amino)-L-phenylalanyl-L-phenylalanyl]amino]-4-hydroxy-6-phenyl-2-(phenylmethyl)hexanoyl]-L-leucyl-L-phenylalanine Amide. Steps A through G were conducted according to the described procedure of Evans, B.E. et al. J. Org. Chem. 50, 4615-4625(1985). Product b was separated into compounds A and B:

A:

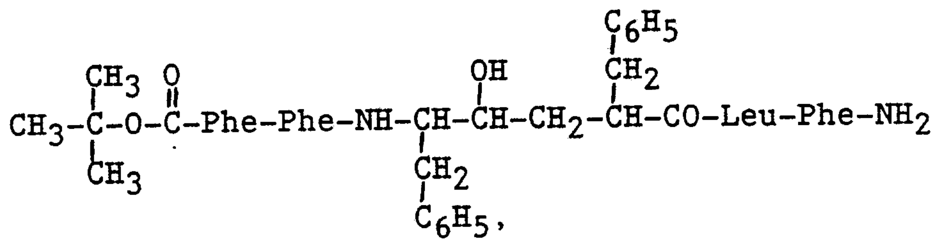

N′-(1,1-Dimethylethoxycarbonyl)-Phe-Phe-5(S)-Amino-4(S)-Hydroxy-6-Phenyl-2(R or S)-(Phenylmethyl)Hexanoyl-Leu-Phe-Amide;

B:

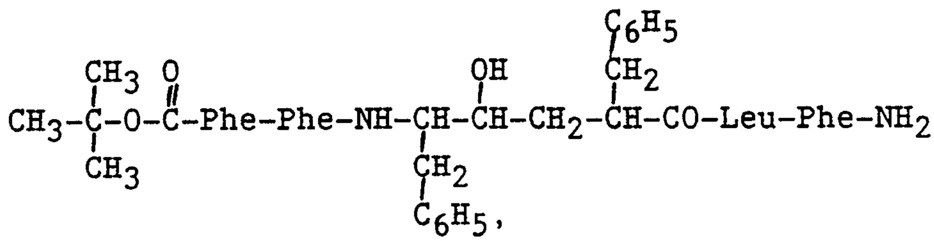

N′-(1,1-Dimethylethoxycarbonyl)-Phe-Phe-5(S)-Amino-4(S)-Hydroxy-6-Phenyl-2(S or R)-(Phenylmethyl)Hexanoyl-Leu-Phe-Amide.

## EXAMPLE 2

Preparation of Recombinant Substrate, Recombinant Protease, and Assay for Inhibition of the Protease

The HIV gag p55 substrate was expressed by recombinant DNA methods in Saccharomyces cerevisiae cells using a 1543 bp gene fragment of the HIV NY5 gag gene that coded for the entire 55 kd gag reading frame placed behind a glyceraldehyde-3-phosphate dehydrogenase promoter. An extract of the cells was made by breaking the cells by pressure induced lysis in breaking buffer (0.1 M HEPES pH 7.5, 10 mM EDTA, 10 mM benzamidine, 1 µg/ml pepstatin A, 0.13 TIU/ml aprotonin, 2mM PMSF (phenylmethylsulfonyl fluoride), 0.1mM E-64 [trans-epoxysuccinyl-2-leucylamido- (4-guanidino)-butane] followed by removal of membranes and cellular debris with a 14,000 x g centrifugation at 4° C for 45 min. The supernatant (16-20 mg/ml protein) was frozen at -70° C. The thawed material was centrifuged at 15,000 g at 4° C for 30 min and the pellet resuspended in 2 original volumes of 50 mM MES (2-[N-morpholino)-ethane sulfonic acid) pH 5.5 and 5% TRITON X-100. The gag p55 was pelleted at 15,000 x g for 30 min at 4° C and resuspended in original volume of pH 5.5 MES buffer, to yield a gag p55 suspension.

To prepare recombinant protease, the HIV protease was expressed in Saccharomyces cerevisae cells by recombinant DNA methods using a gene fusion of the filled in NlaIV to Bal I fragment of the pol gene of the HIV virus (NY5 isolate) ligated onto the filled-in BamH I cleaved alpha mating factor expression vector pJC197 (Gene, 54, pp 113-123 (1987)). The recombinant protease-containing yeast extracts were made by resuspending cells isolated from 5 ml of the yeast culture grown to an $A_{600}=2$, in 0.3 ml breaking buffer (0.1M HEPES (N-2-Hydroxyethylpiperazine-N′-2-ethane sulfonic acid) pH 7.5, 10 mM EDTA, 10 mM benzamidine, 10

μg/ml aprotonin, 10 μg/ml alpha macroglobulin and 0.1 mM E-64, Boehringer Mannheim). The cells were broken by vortexing with an equal volume of glass beads for 3X1 minute intervals followed by centrifugation of the extract for 5 minutes at 4°C at 10,000xg. To assay for protease activity, 5 μL of the yeast extract was added to a 15 μL suspension containing 4 μL of the gag p55 suspension (1 mg protein/mL), in final concentrations of 50 mM MES, (2-[N-Morpholino]-ethane sulfonic acid) pH 5.5, and 40 mM NaCl.
Inhibitors at various concentrations in DMSO (1 μL) were diluted into the 15 μL gag p55 suspension prior to addition of the 5 μL yeast extract containing the protease. Reactions were incubated at 37°C for 1 hr and quenched with 10 μL of SDS loading buffer. Reaction samples were analyzed by electrophoresing on a 16% SDS-PAGE gel followed by electro-transfering onto nitrocellulose membranes. Products were detected by immunoblotting first with murine anti p24 and p17/15 monoclonal antibodies and then with $^{125}I$ antimouse antisera (Amersham) followed by autoradiography. In the absence of drug the levels of gag p55 decreased 10 fold and bands corresponding to gag p17 and gag p24 were visible. At 1 μM of compound B, the sample appeared as the no enzyme control with unprocessed gag 55 and the absence of the gag 17 and gag 24 bands.

## EXAMPLE 3

### Assay for Inhibition of Synthetic Viral Protease

Inhibition studies of the reaction of the synthetic protease [amino acid residues 69-167 of the pol open reading frame in Ratner, L. et al, Nature, 313, 277 (1985) and synthesized by Merrifield solid-phase synthesis] with a peptide substrate [Val-Ser-Gln-Asn-Tyr-Pro-Ile-Val, 2 mg/mL when the reaction is initiated] were in 50 mM Na acetate, pH 5.5, at 30°C for 1 hr. Various concentrations of inhibitor in 1.0 μl DMSO were added to 36 μl of assay solution and the reaction initiated by the addition of 4 ul (1.6 μg) of synthetic protease. The reaction was quenched with 160 ul of 12% acetic acid. Products of the reaction were separated by HPLC (VYDAC wide pore 5 cm C-18 reverse phase, acetonitrile gradient, 0.1% trifluoroacetic acid). The extent of inhibition of the reaction was determined from the peak heights of the products. HPLC of the products, independently synthesized, provided quantitation standards and confirmation of the product composition. Testing of Compounds A and B showed the following results.

| Inh. Conc. | PERCENT INHIBITION Cmpd B | Cmpd A |
|---|---|---|
| 100 μM | 100 | 100 |
| 10 | 100 | 100 |
| 1 | 100 | 40 |
| 0.1 | 100 | 20 |
| 0.01 | 60 | 0 |
| 0.001 | 20 | N.D. |

## EXAMPLE 4

### Inhibition of Beta-Casein Hydrolysis

Inhibition of the synthetic viral protease reaction, of Example 2, with $^{14}C$ - beta-casein as substrate in lieu of peptide substrates, was performed. Beta-casein (Sigma) was dialysed overnight at 4°C in 40 mM $KPO_4$, pH 7.0. Four microliters of the substrate (0.31 mg/ml) are used in each 40 μl reaction. After reaction with the protease for 4 hours, 10 μl of 10% bovine serum albumin and 500 μl of 10% trichloroacetic acid are added, the sample placed on ice for 30 min and centrifuged for 10 min at 15,000 g. From the supernatant a 300 μl aliquot is withdrawn and diluted into 7 ml liquid scintillation cocktail. Typical background level of TCA soluble counts (no enzyme added) is 200 and no inhibitor samples give a net signal of 1000 cpm. Results are as follows:

| Inh. Conc. | PERCENT INHIBITION Cmpd B | Cmpd A |
|---|---|---|
| 100 μM | 60 | 97 |
| 10 | 91 | 65 |
| 1 | 95 | 82 |
| 0.1 | 97 | 14 |
| 0.01 | 67 | 0 |
| 0.001 | 20 | N.D. |

## EXAMPLE 5

Effect of Compound B on HIV gag protein processing

This study was conducted with H9 T-lymphoid cells persistently infected with the HTLV-IIIB isolate of the human immunodeficiency virus (HIV). Individual cultures of the cells, each containing 1.0 x $10^6$ cells, were washed in methionine-free RPMI-1640 medium with 10% dialysed fetal bovine serum. The cells were then resuspended in 0.5 ml of medium containing either no drug or a 50 mcM concentration of Compound B. Following 16 hours of incubation at 37°C, 100 mcCi of $^{35}S$-met was added to each culture.

After an additional 15 min of incubation at 37°C, the cells were washed with chase medium (RPMI-1640 containing a 20-fold excess of unlabeled met and 10% fetal bovine serum). Incubation continued in the presence of the chase medium for 8 hrs, at which time the cells and culture medium supernatants were individually harvested. The cell samples were disrupted by boiling in detergent-containing buffer and, along with the medium supernatants, were immunoprecipitated with human anti-HIV serum. The precipitated proteins were processed by SDS-polyacrylamide gel electrophoresis and were visualized by autoradiography. Treatment of the persistently infected cells with the drug led to accumulation of the p55 gag precursor, as well as two processing intermediates of 49 and 41 kDa, relative to the p24 product. These processing intermediates and p55 were incorporated into newly budded virions as judged by immunoprecipitation from the culture medium supernatants. The untreated control cultures exhibited the expected levels of p24, both within the cells and within the budded virions.

## EXAMPLE 6

Effect of Compound B on HIV spread in cell culture

Cultures of MT-4 T-lymphoid cells were infected with the HTLV-IIIB isolate of HIV at a multiplicity of infection (MOI) of 0.001 infectious virus units per cell. About 25.0 mcM of Compound B was added to some of the cultures 1 hr prior to infection. The cells were incubated at 37°C for 96 hrs. An equal volume of fresh medium (RPMI-1640 with 10% fetal bovine serum) was added to each culture at 48 hrs, post-infection. Fresh drug was added to the drug-treated cells at this time. At the end of the 96 hr incubation, cells from each culture were absorbed onto glass microscope slides. The cells were fixed with methanol/acetone (50/50) at -20°C for 10 min. The fixed cells were then probed for the presence of HIV-specific proteins by immunofluorescence using a human anti-HIV serum. The stained slides were visualized by epifluorescence microscopy. The cells from the untreated, infected cultures exhibited positive fluorescence in $>90\%$ of the cells. In contrast, the drug-treated cultures contained only a 20% level of fluorescing cells.

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims and its equivalents.

## Claims

1. The use of an inhibitor of renin for the preparation of a medicament useful for the treatment of AIDS, or in the prevention or treatment of infection by HIV.

2. The use of renin inhibitors having substituent G, for the preparation of a medicament useful for the inhibition of HIV protease, the treatment of AIDS or the prevention or treatment of infection by HIV; wherein

G is 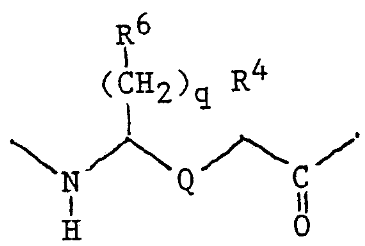 where q is 1 to 4;

$R^4$ is hydrogen; or 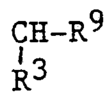, where

$R^3$ is hydrogen; $C_{1-4}$-alkyl; aryl; aryl $C_{1-4}$-alkyl; $C_{1-4}$-alkyl or aryl or aryl $C_{1-4}$-alkyl substituted with up to three members selected from the group consisting of $C_{1-4}$-alkyl, carboxy, hydroxy, $C_{1-4}$-alkoxy, and halo; or indolyl;
$R^9$ is hydrogen; $C_{1-4}$-alkyl; hydroxy; carboxy; or $C_{3-7}$-cycloalkyl;
$R^6$ is $C_{3-6}$-alkyl; $C_{3-7}$-cycloalkyl; aryl; or $C_{3-7}$-cycloalkyl or aryl substituted with up to three members selected from the group consisting of $C_{1-4}$-alkyl, hydroxy, $C_{1-4}$-alkoxy, and halo; and

Q is 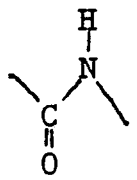 ; 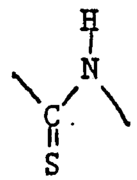 ; 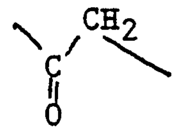 ;

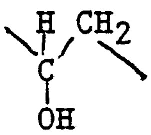 ; 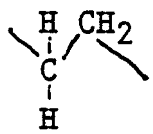 ; 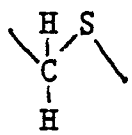 ;

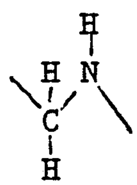 ; 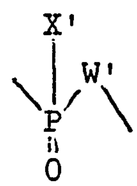 ; 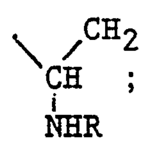 ; 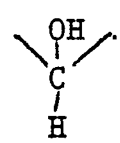 ;

wherein X′ is hydroxy, amino, or mono-or di-$C_{1-4}$-alkyl amino; and W′ is absent; or $-CH_2-$; or

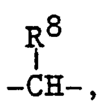,

where $R^8$ is hydrogen or $C_{1-3}$-alkyl;

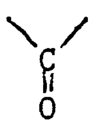 ; 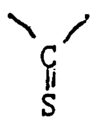 ; or 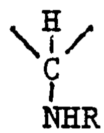 ;

where R is hydrogen; $C_{1-4}$-alkyl; or formyl;
or pharmaceutically acceptable salts thereof.

3. The use of compounds of a formula for the preparation of a medicament useful for the treatment of AIDS, the prevention of infection by HIV, the treatment of infection by HIV or the inhibition of HIV protease, said formula defined as

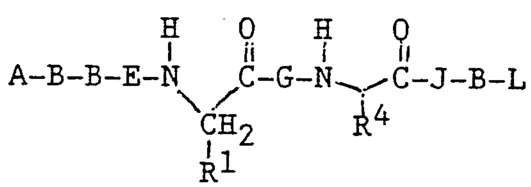

wherein:
A is hydrogen; or

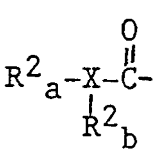

where
X is -O-; -S-; -CH-; -NH-CH-; and $R^2_a$ and $R^2_b$ may be the same or different and are hydrogen; $C_{1-4}$-alkyl; aryl; $C_{3-7}$-cycloalkyl; or $C_{3-7}$-cycloalkyl or aryl substituted with up to five members independently selected from the group consisting of $C_{1-8}$-alkyl, trifluoromethyl, hydroxy, $C_{1-4}$-alkoxy, and halo; except that where X is -O-, only one of $R^2_a$ or $R^2_b$ is present;
B is absent; glycyl; prolyl; sarcosyl; or

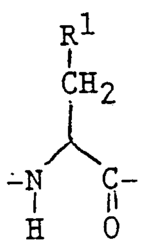

,

where $R^1$ is hydrogen; $-COHN^2$; -COOH $C_{1-4}$-alkyl; hydroxy $C_{1-4}$-alkyl; $C_{1-4}$alkyl substituted with carboxy; aryl; aryl substituted with up to three members selected from the group consisting of $C_{1-4}$-alkyl, hydroxy, $C_{1-4}$-alkoxy, and halo; indolyl; imidazolyl; amino $C_{2-4}$-alkyl;
E is absent; or

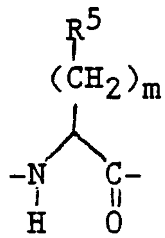

,

where m is 1 to 4; and
$R^5$ is (1) hydrogen;
(2) $C_{1-4}$-alkyl;
(3) $C_{1-4}$-alkyl substituted with carboxy;
(4) aryl;
(5) or aryl substituted with up to three members selected from the group consisting of $C_{1-4}$-alkyl, hydroxy, $C_{1-4}$-alkoxy, and halo;
(6) $R^5$ is indolyl; or
(7) imidazolyl;

G is 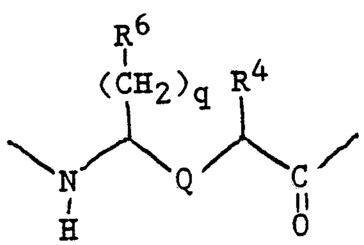

where q is 1 to 4;
$R^4$ is hydrogen; or

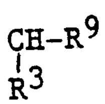

,

where
$R^3$ is hydrogen; $C_{1-4}$-alkyl; aryl; aryl $C_{1-4}$-alkyl; $C_{1-4}$-alkyl or aryl or aryl $C_{1-4}$-alkyl substituted with up to three members selected from the group consisting of $C_{1-4}$-alkyl, carboxy, hydroxy, $C_{1-4}$-alkoxy, and halo; or indolyl;
$R^9$ is hydrogen; $C_{1-4}$-alkyl; hydroxy; carboxy; or $C_{3-7}$-cycloalkyl;
$R^6$ is $C_{3-6}$-alkyl; $C_{3-7}$-cycloalkyl; aryl; or $C_{3-7}$-cycloalkyl or aryl substituted with up to three members selected from the group consisting of $C_{1-4}$alkyl, hydroxy, $C_{1-4}$-alkoxy, and halo; and

Q is 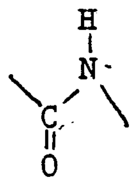 ; 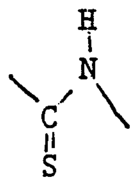 ; 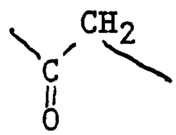 ;

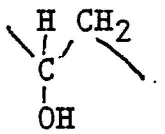 ; 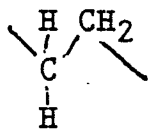 ; 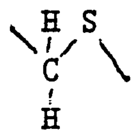 ;

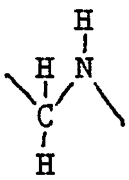 ; 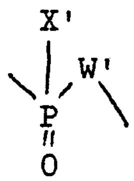 ; 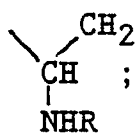 ; 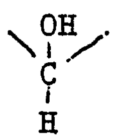 ;

wherein X′ is hydroxy, amino, or mono-or di-$C_{1-4}$-alkyl amino; and W′ is absent, or $-CH_2-$; or

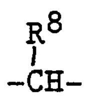,

where $R^8$ is hydrogen or $C_{1-3}$-alkyl;

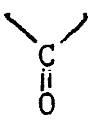 ; 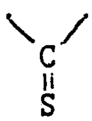 ; or 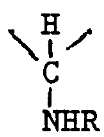 ;

where R is hydrogen; $C_{1-4}$-alkyl; or formyl;

J is absent; or

(1) $-Y-(CH_2)_n-R^7$

where

Y is -NH-;

n is 0 to 5; and

$R^7$ is hydrogen; hydroxy; $C_{1-4}$-alkyl; $C_{3-7}$-cycloalkyl; carbocyclic, aryl; carbocyclic or aryl substituted with up to five members independently selected from the group consisting of $C_{1-6}$-alkyl, hydroxy, $C_{1-4}$-alkoxy, amino, mono- or di-$C_{1-4}$-alkylamino, and halo; amino; mono-, di-, or tri-$C_{1-4}$-alkylamino; amino substituted with heterocycle; guanidyl; heterocycle; monoalkylamino or heterocycle substituted with up to five members independently selected from the group consisting of $C_{1-6}$-alkyl, hydroxy, $C_{1-4}$-alkoxy, halo, amino, and mono- or di-$C_{1-4}$-alkylamino;

(2) 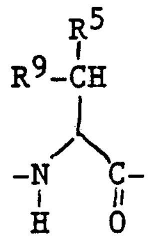 , where $R^5$ and R9 are defined above; or

(3) (a) 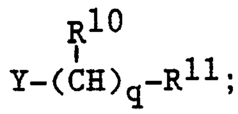; (b) 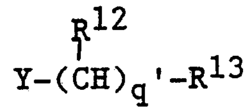; or

(c) 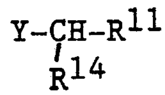

where
Y is -NH-;
q is 1-5;
q′ is 0-5;
$R^{10}$ is hydrogen; hydroxy; $N(R')_2$, where R′ may be the same or different and is hydrogen or $C_{1-4}$-alkyl; guanidyl; or $N(R')_3 \oplus A \ominus$, where R′ is as defined above, and $A\ominus$ is a counterion; provided that at least one $R^{10}$ is not hydrogen;
$R^{11}$ is $C_{1-4}$-alkyl; $C_{3-7}$-cycloalkyl; aryl; aryl substituted with up to three members independently selected from the group consisting of $C_{1-6}$-alkyl, hydroxy, $C_{1-4}$-alkoxy, amino, mono- or di-$C_{1-4}$-alkylamino, amino $C_{1-4}$-alkyl, mono-, di-, or tri-$C_{1-4}$-alkylamino-$C_{1-4}$-alkyl, carboxy, carboxy ester or amide, carboxy-$C_{1-4}$-alkyl, carboxy-$C_{1-4}$-alkyl ester or amide; carboxy, ester or amide; sulfo; heterocycle; or heterocycle substituted with up to five members independently selected from the group consisting of hydroxy, $C_{1-4}$-alkoxy, halo, amino, and mono- or di-$C_{1-4}$-alkylamino;
$R^{12}$ is hydrogen; or carboxy, ester or amide;
$R^{13}$ is carboxy, ester or amide; sulfo; or aryl substituted with up to three members selected from the group consisting of amino-$C_{1-4}$-alkyl, mono-, di-, or tri-$C_{1-4}$-alkylamino-$C_{1-4}$-alkyl, carboxy, carboxy ester or amide,
$R^{14}$ is carboxy, ester or amide,
L is absent; OR″; NHR″; or $N(R'')_2$, where R″ may be the same or different and is hydrogen or $C_{1-4}$-alkyl; or pharmaceutically acceptable salts thereof.

4. The use as claimed in Claim 3, wherein:
A is t-butyloxycarbonyl, ethoxycarbonyl, or t-butylacetyl;
B is absent;
$R^1$ is $C_{1-4}$-alkyl, hydroxy $C_{1-4}$-alkyl, or aryl;
$R^5$ is $C_{1-4}$alkyl or aryl.
$R^6$ is $C_{3-7}$-cycloalkyl, aryl, or hydroxyaryl.

Q is 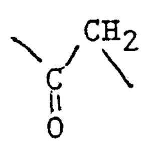 ; 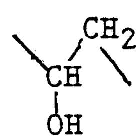 ; 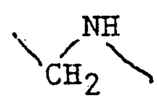 ;

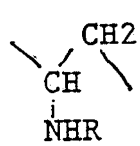 ; or 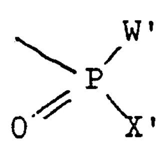 ;

J is defined as in (1) or (2);
$R^7$ is $C_{1-4}$-alkyl, aryl, pyridyl, pyridylamino;
$R^9$ is H;
L is NHR″; and
$R^3$ is $C_{1-4}$-alkyl, aryl, or substituted aryl.

5. The use as claimed in Claim 3, wherein:
A is t-butyloxycarbonyl;
$R^1$ is aryl;
$R^5$ is aryl;

$R^6$ is $C_{3-7}$-cycloalkyl, aryl, or hydroxy aryl;

Q is 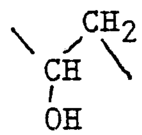 or 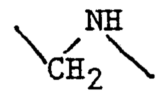 or 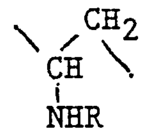 ;

$R^3$ is aryl.

6. The compound N′-(1,1-Dimethylethoxycarbonyl)-Phe-Phe-5(S)-Amino-4(S)-Hydroxy-6-Phenyl-2(R or S)-(Phenylmethyl)-hexanoyl-Leu-Phe-Amide,
or pharmaceutically acceptable salts thereof.

7. The compound N′-(1,1-Dimethylethoxycarbonyl)-Phe-Phe-5(S)-Amino-4(S)-Hydroxy-6-Phenyl-2(S or R)-(Phenylmethyl)-hexanoyl-Leu-Phe-Amide,
or pharmaceutically acceptable salts thereof.

8. The compound N′-Acetyl-Pro-Phe-His-4(S)-Amino-3(S)-Hydroxy-6-methylheptanoyl-Glu-Phe-Gly-Amide, or pharmaceutically acceptable salts thereof.

9. The compound N′-(1,1-dimethylethoxy carbonyl)-Phe-Phe-5(S)-amino-4(S)-hydroxy-6-phenyl-2(R)-(phenylmethyl)hexanoyl-N-(phenylmethyl)-leu-amide,
N′-(1,1-Dimethylethoxycarbonyl)-Phe-Phe-5(S)-amino-4(S)-hydroxy-6-phenyl-2(R)-(phenylmethyl)hexanoyl-Leu-amide,
N′-(1,1-Dimethylethoxycarbonyl)-Asn-5(S)-amino-4(S)-hydroxy-6-phenyl-2(R)-(phenylmethyl)hexanoyl-Leu-Phe-amide,
Asn-5(S)-amino-4(S)-hydroxy-6-phenyl-2(R)-(phenylmethyl)hexanoyl-Leu-Phe-amide, or
N′-(1,1-Dimethylethoxycarbonyl)-Asp-5(S)-amino-4(S)-hydroxy-6-phenyl-2(R)-(phenylmethyl)hexanoyl-N-phenylmethyl)-Ile-amide,
5(S)-[(1,1-dimethylethoxycarbonyl)-Asp]amine-4(S)-hydroxy-6-phenyl-2(R)-(phenylmethyl)-N-(cis-2(R)-hydroxy-1(S)-indanyl)hexanamide,
5(S)-Asp-amino-4(S)-hydroxy-6-phenyl-2(R)-(phenylmethyl-N-(cis-2(R)-hydroxy-1(S)-indanyl)hexanamide,
or pharmaceutically acceptable salt thereof.

10. A pharmaceutical composition comprising the compound of Claim 6 and a pharmaceutical carrier.

11. A pharmaceutical composition comprising the compound of Claim 7 and a pharmaceutical carrier.

12. A pharmaceutical composition comprising the compound of Claim 8 and a pharmaceutical carrier.

13. A pharmaceutical composition comprising any compound of Claim 9 and a pharmaceutical carrier.

14. A compound as defined in any one of claims 1-9 in combination with any of the antivirals, immunomodulators, antibiotics or vaccines of Table 1.

15. The use of the combination as claimed in Claim 14 for the preparation of a medicament useful for the treatment of AIDS, the prevention of infection by HIV, the treatment of infection by HIV, or the inhibition of HIV protease.

16. The use of a compound as claimed in any one of Claims 6, 7, 8 or 9 for the preparation of a medicament useful for the treatment of AIDS, the prevention of infection by HIV, the treatment of infection by HIV, or the inhibition of HIV protease.

**Claims for the following Contracting State: ES**

1.- A process for preparing renin inhibitors useful for the treatment of AIDS, in the prevention of infection by HIV, in the treatment of infection by HIV or the inhibition of HIV protease, said renin inhibitors having formula I

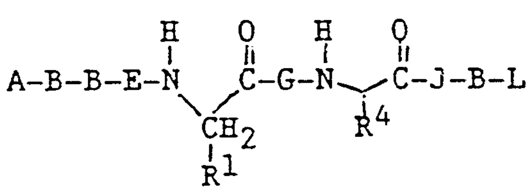

wherein:

A is hydrogen; or 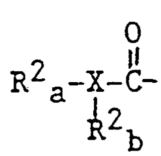

where:

X is -O-; -S-; -CH-; -NH-CH-; and $R^2_a$ and $R^2_b$ may be the same or different and are hydrogen; $C_{1-4}$-alkyl;

aryl; $C_{3-7}$-cycloalkyl; or $C_{3-7}$-cycloalkyl or aryl substituted with up to five members independently selected from the group consisting of $C_{1-8}$-alkyl, trifluoromethyl, hydroxy, $C_{1-4}$-alkoxy, and halo; except that where X is -O-, only one of $R^2{}_a$ or $R^2{}_b$ is present;

B is absent; glycyl; prolyl; sarcosyl; or

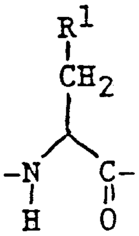,

where $R^1$ is hydrogen; $-COHN^2$; -COOH $C_{1-4}$-alkyl; hydroxy $C_{1-4}$-alkyl; $C_{1-4}$-alkyl substituted with carboxy; aryl; aryl substituted with up to three members selected from the group consisting of $C_{1-4}$-alkyl, hydroxy, $C_{1-4}$-alkoxy, and halo; indolyl; imidazolyl; amino $C_{2-4}$-alkyl;

E is absent; or

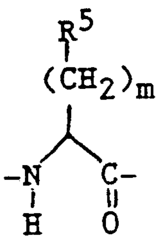,

where m is 1 to 4; and

$R^5$ is (1) hydrogen;

(2) $C_{1-4}$-alkyl;

(3) $C_{1-4}$-alkyl substituted with carboxy;

(4) aryl;

(5) or aryl substituted with up to three members selected from the group consisting of $C_{1-4}$-alkyl, hydroxy, $C_{1-4}$-alkoxy, and halo;

(6) $R^5$ is indolyl; or

(7) imidazolyl;

G is 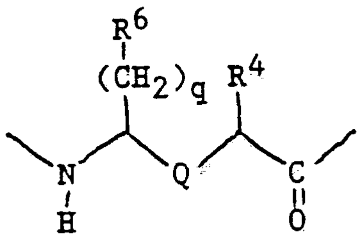

where q is 1 to 4;

$R^4$ is hydrogen; or

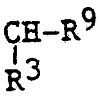,

where

$R^3$ is hydrogen; $C_{1-4}$-alkyl; aryl; aryl $C_{1-4}$-alkyl; $C_{1-4}$-alkyl or aryl or aryl $C_{1-4}$-alkyl substituted with up to three members selected from the group consisting of $C_{1-4}$-alkyl, carboxy, hydroxy, $C_{1-4}$-alkoxy, and halo; or indolyl;

$R^9$ is hydrogen; $C_{1-4}$-alkyl; hydroxy; caraboxy; or $C_{3-7}$-cycloalkyl;

$R^6$ is $C_{3-6}$-alkyl; $C_{3-7}$-cycloalkyl; aryl; or $C_{3-7}$-cycloalkyl or aryl substituted with up to three members selected from the group consisting of $C_{1-4}$-alkyl, hydroxy, $C_{1-4}$-alkoxy, and halo; and

Q is 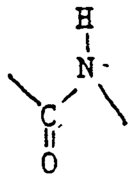 ; 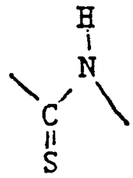 ; 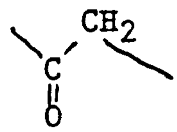 ;

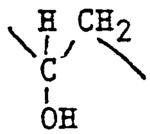 ; 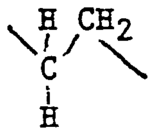 ; 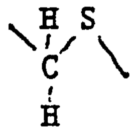 ;

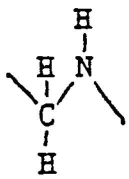 ; 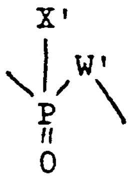 ; 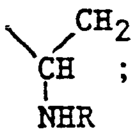 ; 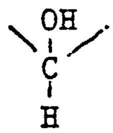 ;

wherein X′ is hydroxy, amino, or mono-or di-$C_{1-4}$-alkyl amino; and W′ is absent, or $-CH_2-$; or

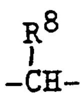 ,

where $R^8$ is hydrogen or $C_{1-3}$-alkyl;

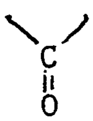 ; 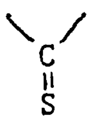 ; or 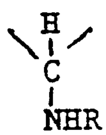 ;

where R is hydrogen; $C_{1-4}$-alkyl; or formyl;
J is absent; or
(1) $-Y-(CH_2)_n-R^7$
where
Y is -NH-;
n is 0 to 5; and
$R^7$ is hydrogen; hydroxy; $C_{1-4}$-alkyl; $C_{3-7}$-cycloalkyl; carbocyclic, aryl; carbocyclic or aryl substituted with up to five members independently selected from the group consisting of $C_{1-6}$-alkyl, hydroxy, $C_{1-4}$-alkoxy, amino, mono- or di-$C_{1-4}$-alkylamino, and halo; amino; mono-, di-, or tri-$C_{1-4}$-alkylamino; amino substituted with heterocycle; guanidyl; heterocycle; monoalkylamino or heterocycle substituted with up to five members independently selected from the group consisting of $C_{1-6}$-alkyl, hydroxy, $C_{1-4}$-alkoxy, halo, amino, and mono- or di-$C_{1-4}$-alkylamino;

(2) 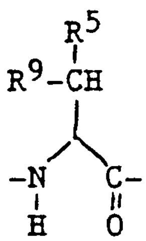 , where $R^5$ and R9 are defined above; or

(3) (a) 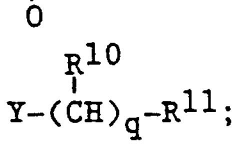 ; (b) 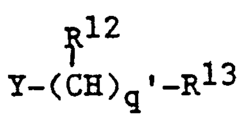 ; or

(c) 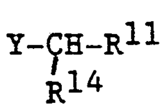

where
Y is -NH-;
q is 1-5;
q′ is 0-5;
$R^{10}$ is hydrogen; hydroxy; $N(R')_2$, where R′ may be the same or different and is hydrogen or $C_{1-4}$-alkyl; guanidyl; or $N(R')_3{\oplus}A{\ominus}$, where R′ is as defined above, and $A\ominus$ is a counterion; provided that at least one $R^{10}$ is not hydrogen;
$R^{11}$ is $C_{1-4}$-alkyl; $C_{3-7}$-cycloalkyl; aryl; aryl substituted with up to three members independently selected from the group consisting of $C_{1-6}$-alkyl, hydroxy, $C_{1-4}$-alkoxy, amino, mono- or di-$C_{1-4}$-alkylamino, amino $C_{1-4}$-alkyl, mono-, di-, or tri-$C_{1-4}$-alkylamino-$C_{1-4}$-alkyl, carboxy, carboxy ester or amide, carboxy-$C_{1-4}$-alkyl, carboxy-$C_{1-4}$-alkyl ester or amide; carboxy, ester or amide; sulfo; heterocycle; or heterocycle substituted with up to five members independently selected from the group consisting of hydroxy, $C_{1-4}$-alkoxy, halo, amino, and mono- or di-$C_{1-4}$-alkylamino;
$R^{12}$ is hydrogen; or carboxy, ester or amide;
$R^{13}$ is carboxy, ester or amide; sulfo; or aryl substituted with up to three members selected from the group consisting of amino-$C_{1-4}$-alkyl, mono-, di-, or tri-$C_{1-4}$-alkylamino-$C_{1-4}$-alkyl, carboxy, carboxy ester or amide,
$R^{14}$ is carboxy, ester or amide,
L is absent; OR″; NHR″; or $N(R'')_2$, where R″ may be the same or different and is hydrogen or $C_{1-4}$-alkyl; or pharmaceutically acceptable salts thereof,
said process being characterized by:

a) forming the desired G component;

b) coupling the other components to both the amine group and the carboxy group of the G component, either one by one or in blocks, each component being duly protected when necessary by protecting groups which are adequate to the particular coupling conditions and to the aminoacids and peptides components involved in the synthesis by forming amide bonds by any coupling method of either solution-phase or solid-phase peptide synthesis; and

c) removing any protecting group which could be present if desired, or if necessary.

2.- The process of claim 1, wherein:
A is t-butyloxycarbonyl, ethoxycarbonyl, or t-butylacetyl;
B is absent;
$R^1$ is $C_{1-4}$-alkyl, hydroxy $C_{1-4}$-alkyl, or aryl;
$R^5$ is $C_{1-4}$alkyl or aryl.
$R^6$ is $C_{3-7}$-cycloalkyl, aryl, or hydroxyaryl.

Q is 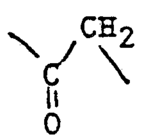 ; 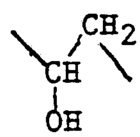 ; 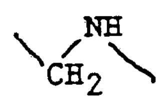 ;

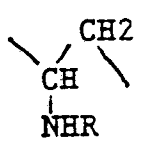 ; or 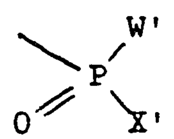 ;

J is defined as in (1) or (2);
$R^7$ is $C_{1-4}$-alkyl, aryl, pyridyl, pyridylamino;
$R^9$ is H;
L is NHR″; and
$R^3$ is $C_{1-4}$-alkyl, aryl, or substituted aryl.

3. The process of claim 1, wherein:
A is t-butyloxycarbonyl;
$R^1$ is aryl;
$R^5$ is aryl;
$R^6$ is $C_{3-7}$-cycloalkyl, aryl, or hydroxy aryl;

Q is 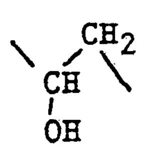 or 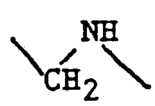 or 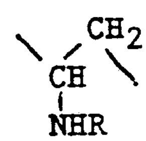 ;

$R^3$ is aryl.

4.- The process of claim 1, wherein the compound obtained N′-(1,1-Dimethylethoxycarbonyl)-Phe-Phe-5(S)-Amino-4(S)-Hydroxy-6-Phenyl-2(R or S)-(Phenylmethyl)-hexanoyl-Leu-Phe-Amide, or pharmaceutically acceptable salts thereof.

5.- The process of claim 1, wherein the compound obtained N′-(1,1-Dimethylethoxycarbonyl)-Phe-Phe-5(S)-Amino-4(S)-Hydroxy-6-Phenyl-2(S or R)-(Phenylmethyl)-hexanoyl-Leu-Phe-Amide, or pharmaceutically acceptable salts thereof.

6.- The process of claim 1, wherein the compound obtained N′-Acetyl-Pro-Phe-His-4(S)-Amino-3(S)-Hydroxy-6-methylheptanoyl-Glu-Phe-Gly-Amide, or pharmaceutically acceptable salts thereof.

7.- The process of claim 1, wherein the compound obtained is selected from N′-(1,1-dimethylethoxy carbonyl)-Phe-Phe-5(S)-amino-4(S)-hydroxy-6-phenyl-2(R)-(phenylmethyl)hexanoyl-N-(phenylmethyl)leu-amide,
N′-(1,1-Dimethylethoxycarbonyl)-Phe-Phe-5(S)-amino-4(S)-hydroxy-6-phenyl-2(R)-(phenylmethyl)hexanoyl-Leu-amide,
N′-(1,1-Dimethylethoxycarbonyl)-Asn-5(S)-amino-4(S)-hydroxy-6-phenyl-2(R)-(phenylmethyl)hexanoyl-Leu-Phe-amide,
Asn-5(S)-amino-4(S)-hydroxy-6-phenyl-2(R)-(phenylmethyl)hexanoyl-Leu-Phe-amide, or
N′-(1,1-Dimethylethoxycarbonyl)-Asp-5(S)-amino-4(S)-hydroxy-6-phenyl-2(R)-(phenylmethyl)hexanoyl-N-phenylmethyl)-Ile-amide,
5(S)-[(1,1-dimethylethoxycarbonyl)-Asp]amine-4(S)-hydroxy-6-phenyl-2(R)-(phenylmethyl)-N-(cis-2(R)-hydroxy-1(S)-indanyl)hexanamide,
5(S)-Asp-amino-4(S)-hydroxy-6-phenyl-2(R)-(phenylmethyl-N-(cis-2(R)-hydroxy-1(S)-indanyl)hexanamide,
or pharmaceutically acceptable salt thereof.